# FASCICULE DE BREVET EUROPEEN

(11) **EP 2 788 495 B1**
(45) Date de publication et mention de la délivrance du brevet: **19.08.2015**
(21) Numéro de dépôt: 12794441.1
(22) Date de dépôt: 06.11.2012
(51) Int. Cl.: C12Q 1/04, G01N 33/68

(54) **PROCEDE DE DETECTION DE L'HEMOLYSINE DELTA DE STAPHYLOCOCCUS AUREUS PAR SPECTROMETRIE DE MASSE DIRECTEMENT A PARTIR D'UNE POPULATION BACTERIENNE**
VERFAHREN ZUR ERKENNUNG VON DELTA-HÄMOLYSIN AUS STAPHYLOCOCCUS AUREUS DURCH MASSENSPEKTROMETRIE DIREKT UNTER VERWENDUNG EINER BAKTERIENPOPULATION
METHOD FOR DETECTING DELTA HAEMOLYSIN OF STAPHYLOCOCCUS AUREUS BY MASS SPECTROMETRY DIRECTLY USING A BACTERIAL POPULATION

(30) Priorité: 08.11.2011 FR 1160176
(43) Date de publication de la demande: 15.10.2014
(73) Titulaire: Biomérieux, 69280 Marcy l'Étoile (FR); Hospices Civils De Lyon, 69229 Lyon Cedex 02 (FR); Université Claude Bernard Lyon I, 69622 Villeurbanne Cedex (FR)
(72) Inventeur: VANDENESCH, François, F-69140 Rillieux La Pape (FR); DAUWALDER, Olivier, F-69008 Lyon (FR); CHARRIER, Jean-Philippe, F-69160 Tassin La Demi-Lune (FR); WELKER, Martin, 10967 Berlin (DE)
(74) Mandataire: Sarlin, Laure V.
(86) Numéro de dépôt international: PCT/FR2012/052556
(87) Numéro de publication internationale: WO 2013/068685

(56) Documents cités:
- SOMERVILLE G A ET AL: "Synthesis and deformylation of Staphylococcus aureus -toxin are linked to tricarboxylic acid cycle activity", JOURNAL OF BACTERIOLOGY, vol. 185, no. 22, 31 octobre 2003 (2003-10-31), pages 6686-6694, XP055031106, ISSN: 0021-9193, DOI: 10.1128/JB.185.22.6686-6694.2003 cité dans la demande
- FOWLER V GET AL: "Persistent bacteremia due to methicillin-resistant Staphyloccus aureus infection is associated with agr dysfunction and low-level in vitro resistance to thrombin-induced platelet microbicidal protein", JOURNAL OF INFECTIOUS DISEASES, vol. 190, no. 6, septembre 2004 (2004-09), pages 1140-1149, XP055031143, ISSN: 0022-1899 cité dans la demande
- SHAH H N ET AL: "Tracing the transition of methicillin resistance in sub-populations of staphylococcus aureus, using SELDI-TOF mass spectrometry and artificial neural network Analysis", SYSTEMATIC AND APPLIED MICROBIOLOGY, vol. 34, no. 1, novembre 2010 (2010-11), pages 81-86, XP028145456, ISSN: 0723-2020, DOI: 10.1016/J.SYAPM.2010.11.002
- JACKSON K A ET AL: "Optimisation of intact cell MALDI method for fingerprinting of methicillin-resistant Staphylococcus aureus", JOURNAL OF MICROBIOLOGICAL METHODS, vol. 62, no. 3, 1 septembre 2005 (2005-09-01), pages 273-284, XP027746420, ISSN: 0167-7012
- FOX K ET AL: "Speciation of coagulase negative staphylococci, isolated from indoor air, using SDS page gel bands of expressed proteins followed by MALDI TOF MS and MALDI TOF-TOF MS-MS analysis of tryptic peptides", JOURNAL OF MICROBIOLOGICAL METHODS, vol. 84, no. 2, 2 décembre 2010 (2010-12-02), pages 243-250, XP028137124, ISSN: 0167-7012, DOI: 10.1016/J.MIMET.2010.12.007

## Description

La présente invention concerne le domaine technique de l'analyse de bactéries de *Staphylococcus aureus*. Hébergé à l'état de portage par 20 à 30% de la population, *Staphylococcus aureus* est une bactérie responsable de colonisations et d'infections (Wertheim, Melles et al. 2005). Deux grands types d'infections peuvent être distingués : les infections suppuratives impliquant des facteurs d'adhésion « *Microbial Surface Component Recognizing Adhesive Matrix Molecules* » ou MSCRAMM (la protéine de liaison du fibrinogène [FnBP], la protéine A, les coagulases, *etc.*) ; et les infections toxiniques requérant la sécrétion d'exotoxines dont les plus connues sont les hémolysines, la leucocidine de Panton et Valentine [PVL], les entérotoxines staphylococciques et les exfoliatines (Otto 2010). Toutes les souches de *S. aureus* ne possèdent pas tous les facteurs de virulences et ces facteurs ne sont pas tous exprimés en même temps au cours de la croissance bactérienne. En phase exponentielle de croissance, il y a expression des facteurs d'adhésion, nécessaires à la colonisation et l'envahissement de l'hôte. A l'inverse, en fin de phase exponentielle de croissance et durant la phase stationnaire, la bactérie exprime majoritairement des toxines nécessaires à la destruction des cellules de l'hôte, source de nutriments essentiels à sa croissance. *S. aureus* possède de nombreux facteurs de régulation qui vont contrôler ces processus (Dufour, Jarraud et al. 2002). Parmi eux, le système « *Accessory Gene Regulator* » [agr] est l'un des systèmes les plus importants.

Le système agr de *S. aureus* régule un grand nombre de gènes de virulence et du métabolisme de la bactérie. Il consiste en un locus de 5 gènes (*agr*BDCA et *hld*) répartis en deux transcrits divergents : ARN II et ARN III. L'ARN II est le messager de l'opéron P2 qui contient les gènes *agr*BDCA ; et l'ARN III contient *hld,* le gène de l'hémolysine Delta [δ]. *agr*C code une histidine kinase et *agr*A est le récepteur du système à deux composants. *agr*D code un peptide, qui est maturé et sécrété par *agr*B. Le peptide résultant est un peptide auto inducteur qui active *agr*C lequel stimule *agr*A. Une fois activé, le récepteur *agr*A induit les transcriptions des ARN II et ARN III grâce aux promoteurs respectifs P2 et P3. L'ARN III contient *hld,* gène codant l'hémolysine δ, mais est surtout un ARN régulateur, véritable effecteur du système agr. Ainsi, cet ARN III régule un grand nombre de gènes tant au niveau transcriptionnel que traductionnel (Verdon, Girardin et al. 2009; Felden, Vandenesch et al. 2011). Les mutations induisant une dysfonction du système agr provoquent des modifications de l'expression des autolysines et des hémolysines ; et des effets sur le phénotype bactérien, impliqués notamment dans le pouvoir pathogène (Schweizer, Furuno et al. 2011).

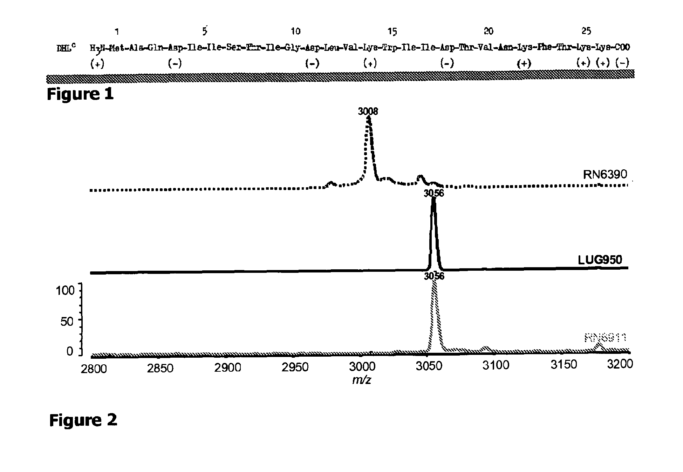

L'hémolysine δ de *S. aureus,* également appelée δ-toxine ou δ-lysine, est codée par le gène *hld* qui appartient au système agr. Il est localisé au sein de l'ARN III, effecteur du système agr. La traduction de l'ARN III en hémolysine δ est retardée (≈1 heure) et intervient en fin de phase exponentielle de croissance (Balaban and Novick 1995; Somerville, Cockayne et al. 2003). L'hémolysine δ est ainsi le reflet de la transcription de l'ARN III donc, de la fonctionnalité du système agr (Felden, Vandenesch et al. 2011). Elle appartient à la famille des toxines hémolytiques de *S. aureus* et crée des pores dans la membrane cellulaire. Elle provoque notamment la lyse des érythrocytes d'humain, de lapin, de mouton, de cheval, de chat, de poulet, *etc.* par destruction des phospholipides des membranes cellulaires (Kreger, Kim et al. 1971). Elle est également impliquée dans la prévention de la formation du biofilm et dans l'initiation du stress oxydatif dans les polynucléaires neutrophiles humains (Somerville, Cockayne et al. 2003). Elle est constituée de 26 acides aminées tel qu'illustré **Figure 1** et possède une masse de 2,9 kDa (Fitton, Dell et al. 1980). Elle est présente sous deux formes: *i)* formylée en N-terminal sur le résidu méthionine, qui est la forme native et majoritaire, dénommée dans ce document hémolysine δ ; et *ii)* déformylée (Verdon, Girardin et al. 2009). La déformylation est assurée par une enzyme : une peptide déformylase dépendante du fer. Durant la phase exponentielle et en phase post exponentielle précoce, l'hémolysine δ est déformylée pour donner une entité faiblement chimio-attractante (Somerville, Cockayne et al. 2003). Au fur et à mesure de la croissance bactérienne, la concentration en fer va être réduite, entraînant une réduction de l'activité de la déformylase. Il y a alors accumulation d'hémolysine δ, douée de propriétés chimio-attractives des polynucléaires neutrophiles ; et inductrice de réponse inflammatoire, source de nutriments pour la future croissance bactérienne (Somerville, Cockayne et al. 2003). Enfin, des polymorphismes ont été décrits : un variant a été retrouvé dans des souches d'origine canine. Il présente des variations en acides aminés ; une digestion réduite à l'action de la trypsine ; un pouvoir hémolytique réduit sur les hématies de mouton et une activité hémolytique réduite à des températures inférieures à 25°C (Turner 1978). Dans les souches d'origine humaine, un polymorphisme de type glycine en position 10 remplacée par une sérine (G10S) est décrit dans Genebank™ (Figueiredo, Jarraud et al. 2000) mais la prévalence de cette mutation n'est pas connue.

Les souches de *S. aureus* déficientes pour le système agr ont une fréquence estimée entre 15% et 22% selon les études (Traber, Lee et al. 2008; Schweizer, Furuno et al. 2011; Tsuji, Maclean et al. 2011). Ces souches sont plus fréquentes parmi les *S. aureus* résistants à la méthicilline [SARM] hospitaliers en comparaison aux SARM communautaires (Tsuji, Maclean et al. 2011). Cependant, des souches déficientes pour agr ont été retrouvées dans tous les fonds génétiques agr : I, II, III et IV, ne supportant pas l'hypothèse clonale (Rose, Rybak et al. 2007).

Ces souches ont une réelle pertinence clinique car isolées au sein de prélèvements biologiques ayant subi le minimum de manipulations et ne sont pas le résultat de mutations survenant lors des repiquages successifs (Traber, Lee et al. 2008). Différents liens cliniques ont été établis entre le dysfonctionnement du système agr et l'absence d'hémolysine δ.

Par exemple, en 2011, une étude rétrospective réalisée entre 2003 et 2007 portant sur 814 bactériémies à *S. aureus* a retrouvé 22% de souches présentant une dysfonction du système agr. Dans 18% des cas, le patient infecté par ces souches décéda dans les 30 jours contre 12% avec des souches dont le système agr est fonctionnel (*p*=0.03). Ainsi, une infection par une souche de *S. aureus* déficiente pour agr était capable de prédire la mortalité avec une sensibilité de 30%, une spécificité de 79%, une valeur prédictive positive de 18 % et une valeur prédictive négative de 88% (Schweizer, Furuno et al. 2011).

Par ailleurs, une étude clinique rétrospective portant sur les bactériémies persistantes (BP) à SARM (*i.e.* persistance d'hémocultures positives à SARM au-delà de 7 jours sous antibiothérapie adaptée) a montré que les souches de SARM présentant un dysfonctionnement du système agr, objectivée par la recherche de l'hémolysine δ sur gélose, étaient plus fréquemment retrouvées au cours des bactériémies persistantes (BP) en comparaison aux bactériémies résolutives (BR) [71,4% des souches du groupes BP *versus* 38,9% des souches du groupe BR ; *p*=0,057). Ainsi, l'absence de production d'hémolysine δ, témoin de la dysfonction du système agr, pourrait être un marqueur de bactériémie persistante, nécessitant une prise en charge accrue (*i.e.* antibiothérapie optimale) (Fowler, Sakoulas et al. 2004).

Un lien avec l'autolyse bactérienne a également été établi. Le système agr contrôle l'expression de nombreux gènes notamment *lytS* et *lytR.* Ainsi, une dysfonction du système agr est associée à la formation d'agrégats en milieu liquide ; des altérations de la surface cellulaire, qui devient rugueuse et diffuse ; et une prédisposition à l'autolyse (Brunskill and Bayles 1996).

Vuong *et al.* ont, quant à eux, montré dans un modèle d'adhésion au polystyrène que 78% des souches déficientes pour agr formaient un biofilm contre seulement 6% des souches exprimant agr. Ce phénomène a également été confirmé par l'ajout d'inhibiteurs d'agr et semble dû aux propriétés surfactantes de l'hémolysine δ (Vuong, Saenz et al. 2000). Cependant, autant le dysfonctionnement du système agr est nécessaire pour la création du biofilm, autant la dispersion du biofilm semble être sous la dépendance du système agr soulignant la coexistence probable de souches déficientes et de souches fonctionnelles pour le système agr au cours des infections chroniques (Boles and Horswill 2008). Cumulés, ces résultats plaident en faveur de la recherche des souches déficientes en agr au cours des infections chroniques ou sur dispositifs intra-vasculaires.

Le lien avec la mucoviscidose a également été établi. En 2000, Goerke *et al.* ont étudié l'expression de l'ARN III dans les souches de *S. aureus* isolées de prélèvements respiratoires de patients atteints de mucoviscidose. Ils ont montré la prédominance des souches déficientes en agr. La fonctionnalité du système agr ne serait donc pas nécessaire à la colonisation et à l'infection au cours de la fibrose kystique (Sakoulas, Eliopoulos et al. 2005).

Enfin, le lien avec le phénotype de résistance aux glycopeptides (*i.e.* GISA pour « *Glycopeptide intermediate Staphylococcus aureus* ») a également été rapporté. L'administration prolongée (*i.e.* supérieure à 9 mois) de vancomycine chez un patient infecté par une souche de SARM présentant un système agr fonctionnel induit une réduction de l'expression de l'hémolysine δ sans que cette dernière soit complètement abolie. A l'arrêt du traitement, une augmentation notable de l'expression de l'hémolysine δ est mesurée (Sakoulas, Gold et al. 2006). Par ailleurs, les souches présentant une dysfonction du système agr montrent une sensibilité réduite à la vancomycine, bien que la pharmacodynamie de cette dernière ne soit pas altérée (Tsuji, Maclean et al. 2011). En 2002, Sakoulas *et al.* ont démontré que l'ensemble de souches incluses dans la collection de GISA de son laboratoire présentait un dysfonctionnement du système agr (Sakoulas, Eliopoulos et al. 2002). En 2005, le lien entre émergence d'un phénotype hétéro GISA et l'exposition prolongée à la vancomycine de souches déficientes pour le système agr a été confirmé. Cette diminution de la sensibilité à la vancomycine est également associée à une réduction de lyse bactérienne et une sensibilité moindre aux peptides anti microbiens plaquettaires (Sakoulas, Eliopoulos et al. 2005). Cette association a également été confirmée par l'étude de Rose *et al.* montrant la capacité accrue des souches déficientes pour agr à accroitre leurs concentrations minimales inhibitrices (CMI) à la vancomycine, devenant ainsi GISA. A l'inverse, ce phénomène n'est pas trouvé avec un autre antibiotique anti *S. aureus* appartenant à une autre classe d'antibiotiques : la daptomycine (Rose, Rybak et al. 2007). Ainsi, la mise en évidence de l'absence de production d'hémolysine δ pourrait être un marqueur précoce de détection de l'évolution vers le phénotype GISA ou hétéro GISA, particulièrement lors d'un traitement par de la vancomycine (Fowler, Sakoulas et al. 2004).

Aussi, compte tenu de l'intérêt clinique que présente la détection de l'hémolysine δ, différentes méthodes de mesure de l'hémolysine δ de *S. aureus* ont été décrites. On peut citer les tests d'hémolyse, les biotests sur modèle animal, la « *Reverse Transcription-Polymerase Chain Reaction* [RT-PCR], le *« Northern blot* », le séquençage et la chromatographie haute performance [HPLC].

Dans les tests d'hémolyse, l'hémolysine δ peut être détectée par la mesure de l'hémolyse induite par une souche de *S. aureus* cultivée sur gélose au sang en présence d'anticorps anti hémolysine Alpha [α] et Bêta [β]. L'hémolysine δ peut également être détectée par la recherche de la synergie d'hémolyse entre l'hémolysine δ et l'hémolysine β (Verdon, Girardin et al. 2009). Différentes méthodologies ont été décrites : Sakoulas *et al.* détectent l'hémolysine δ en utilisant la souche de *S. aureus* RN4420 productrice d'une large zone d'hémolyse induite par l'hémolysine β. Une méthodologie dérivée a également été décrite par Schweitzer *et al.* Ces méthodes sont faciles à effectuer mais nécessitent la réalisation d'un test « dédié » et présentent une lecture subjective : la recherche d'une synergie d'hémolyse, est parfois difficile à observer. Enfin, ces tests peuvent être entachés de faux négatifs en comparaison au *« Northern Blot* » et au séquençage du gène *agr* (Traber, Lee et al. 2008).

Historiquement, des biotests sur modèle animal ont également été utilisés. Dans ce cas, l'activité de l'hémolysine δ peut être détectée par la mesure de la dose létale minimale [DLM] sur des souris ou des cobayes. Elle nécessite cependant des doses importantes (DLM souris = 110 mg/kg et cobaye = 30 mg/kg). Une autre méthodologie consiste à rechercher la capacité de l'hémolysine δ à induire une nécrose cutanée chez le lapin. Environ 1 mg d'hémolysine δ induit 24h plus tard, un large érythème (>28mm de diamètre) et une induration suivie à J+3 d'une zone nécrotique. Une desquamation peut également être observée, à dose plus faible (Kreger, Kim et al. 1971).

L'expression de l'hémolysine δ peut également être évaluée par RT-PCR ciblant l'ARN III. A partir d'une souche, l'ARN total est extrait et une RT-PCR compétitive est réalisée. Afin de normaliser les résultats, Goerke *et al.* ont utilisé le gène de ménage codant la gyrase (*gyr*) dont l'expression est constante quelles que soient les phases de croissance. La RT-PCR ARN III représente une technique sensible, spécifique et quantitative. Cependant, sa réalisation est réservée à des laboratoires spécialisés en raison d'un risque élevé de contaminations (Goerke, Campana et al. 2000).

L'expression de l'hémolysine δ peut être détectée indirectement par la réalisation d'un « *Northern Blot* » ciblant l'ARN III (Kornblum, Projan et al. 1988; Goerke, Campana et al. 2000; Traber and Novick 2006).

Certains auteurs ont également utilisé le séquençage complet du locus agr pour identifier des mutations ponctuelles pouvant expliquer son dysfonctionnement (Traber and Novick 2006).

Plusieurs techniques HPLC ont également été utilisées. En 2000, Otto et Gotz ont décrit une méthode qualitative et quantitative de mesure de l'hémolysine δ par chromatographie liquide haute performance (HPLC). Les surnageants de culture bactériennes réalisées en bouillon trypticase soja sont centrifugés à 19 000g pendant 5 minutes. Les surnageants sont ensuite analysés par HPLC sur une colonne PHE (« *Phenyl derivated* ») de 1mL puis élués par un gradient de 10 à 90% de solvant B, le solvant A étant un mélange 0,1% acide trifluoroacétique [TFA]/eau et le solvant B étant un mélange 0,1%TFA/acétonitrile. La détection est réalisée par un spectromètre UV à barrettes de diodes à 214 ou 280 nm (Otto and Gotz 2000). En 2002, Somerville *et al*. ont, quant à eux, décrit une méthodologie proche de celle d'Otto et Gotz de détection et de mesure de l'hémolysine δ par HPLC mais couplée à une détection par spectrométrie de masse avec une source électrospray. Ils obtiennent ainsi une m/z à 3007 Thomson [Th] et à 2972 Th pour les formes natives et déformylées de l'hémolysine δ (Somerville, Cockayne et al. 2003).

Dans ce contexte, les inventeurs se sont intéressés à un nouveau procédé d'étude de colonies de *Staphylococcus aureus* qui permette d'émettre un diagnostic et/ou d'établir un lien avec la présence ou l'absence d'hémolysine δ. Ce procédé se doit d'être rapide et aisé d'utilisation, et notamment ne pas nécessiter des étapes lourdes d'extraction de protéines.

La présente invention concerne un procédé d'étude d'un échantillon contenant une population bactérienne de *Staphylococcus aureus* par une technique de spectrométrie de masse comprenant les étapes suivantes :
a) Disposer l'échantillon en contact avec un milieu permettant l'ionisation par action d'un rayon laser des molécules présentes dans l'échantillon,
b) Ioniser les molécules présentes dans l'échantillon grâce à un rayon laser,
c) Accélérer les molécules ionisées obtenues grâce à une différence de potentiel,
d) Laisser se déplacer librement les molécules ionisées et accélérées dans au moins un tube sous pression réduite,
e) Détecter au moins une partie des molécules ionisées et accélérées s'étant déplacées librement, de manière à mesurer le temps qu'elles ont mis pour parcourir au moins un tube sous pression réduite et à obtenir un signal correspondant au nombre de molécules ionisées détectées à un instant donné,
f) Calculer le rapport masse sur charge [m/z] des molécules détectées, de manière à obtenir un signal correspondant au nombre de molécules ionisées d'une même masse sur charge [m/z], en fonction du rapport m/z des molécules détectées,
g) Déterminer, de manière directe ou indirecte, si parmi les molécules ionisées obtenues à l'étape b), il y avait ou pas des molécules ionisées de masse/charge [m/z] égale à 3005 ± 5 Th ou égale à 3035 ± 5 Th,
h) Emettre une décision conditionnée par le résultat obtenu à l'étape g).

Selon une première variante de mise en oeuvre, l'étape h) correspond à une étape de corrélation de l'absence à la fois de molécules ionisées de m/z égale à 3005 ± 5 Th et de molécules ionisées de m/z égale à 3035 ± 5 Th avec l'absence d'hémolysine δ et de son variant G10S dans la population bactérienne de *Staphylococcus aureus* analysée.

L'hémolysine δ de séquence **SEQ ID N°1** : XaaAQDIISTIGDLVKWIIDTVNKFTKK, avec Xaa = méthionine formylée en position N-terminale, a une masse monoisotopique protonée (MH⁺) de 3005,6 Th.

Son variant de séquence **SEQ ID N°2** : XaaAQDIISTISDLVKWIIDTVNKFTKK, avec Xaa = méthionine formylée en position N-terminale, a une masse monoisotopique protonée (MH⁺) de 3035,6 Th.

Selon une deuxième variante de mise en oeuvre, l'étape h) correspond à une étape de corrélation de l'absence à la fois de molécules ionisées de m/z égale à 3005 ± 5 Th et de molécules ionisées de m/z égale à 3035 ± 5 Th avec un dysfonctionnement du système agr (« *Accessory Gene Regulator* ») chez la population bactérienne de *Staphylococcus aureus* analysée.

Selon une troisième variante de mise en oeuvre, la population bactérienne de *Staphylococcus aureus* analysée provient d'un échantillon biologique d'un patient et l'étape h) correspond à une étape de corrélation de l'absence à la fois de molécules ionisées de m/z égale à 3005 ± 5 Th et de molécules ionisées de m/z égale à 3035 ± 5 Th avec l'émission pour ledit patient, d'un diagnostic clinique connu pour être lié au dysfonctionnement du système agr.

Selon une quatrième variante de mise en oeuvre, la population bactérienne de *Staphylococcus aureus* analysée provient d'un échantillon biologique d'un patient et l'étape h) correspond à une étape de corrélation de l'absence à la fois de molécules ionisées de m/z égale à 3005 ± 5 Th et de molécules ionisées de m/z égale à 3035 ± 5 Th avec le diagnostic d'une infection chronique chez le patient. Une infection chronique est définie sur la base du critère clinico-biologique consistant à retrouver l'isolement, jusqu'à 6 mois auparavant, d'une souche de *S. aureus* présentant le même antibiogramme que la souche isolée lors de l'étude. A titre d'exemples de telles infections chroniques, on peut citer les infections ostéoarticulaires, les infections chroniques du pied diabétique, les infections sur dispositif vasculaire implantable.

Selon une cinquième variante de mise en oeuvre, l'étape h) correspond à une étape de corrélation de l'absence à la fois de molécules ionisées de m/z égale à 3005 ± 5 Th et de molécules ionisées de m/z égale à 3035 ± 5 Th avec un classement de la population bactérienne *Staphylococcus aureus* analysée comme risquant de présenter une sensibilité diminuée aux glycopeptides (*i.e.* GISA).

L'échantillon sur lequel le procédé de l'invention peut être mis en oeuvre peut être d'origine biologique, notamment animale ou humaine. Il peut alors correspondre à un prélèvement de fluide biologique, par exemple sang total, sérum, plasma, urine, liquide céphalo-rachidien, sécrétion organique, à un prélèvement tissulaire ou à des cellules isolées. Ce prélèvement peut être utilisé tel quel, ou bien il peut subir préalablement à l'étude, une préparation de type enrichissement ou culture, selon des méthodes connues de l'homme du métier.

Dans le cadre de l'invention, l'échantillon à étudier correspond à un milieu cellulaire comprenant une population bactérienne, et non à une ou plusieurs protéines obtenues après une étape d'extraction ou de purification. De préférence, l'échantillon étudié contient une population bactérienne, c'est-à-dire qu'il contient au moins 10 bactéries.

La détection peut être réalisée, notamment à partir du bouillon d'une culture bactérienne, issue d'un échantillon biologique, ou à partir d'une hémoculture. Le plus souvent, le spectre de masse est obtenu à partir d'un échantillon obtenu à partir d'une culture bactérienne sur gélose.

Le milieu cellulaire est mis en suspension directement dans ou sur la matrice. Une telle technique est connue sous le nom de spectrométrie de masse sur cellules entières (WC-MS, de l'anglais « *whole-cell mass spectrometry* ») et peut être menée sans procédure d'extraction préalable, fastidieuse et consommatrice en temps. Cette technique a déjà été largement utilisée par le passé pour l'identification bactérienne, mais son utilisation dans le cas d'identification de protéines est très limitée, compte tenu de la complexité de l'échantillon. Dans la majorité des cas, l'assignement des protéines correspondantes aux profils obtenus par WC-MS est inconnu ou ambigu. Seuls sont comparés les profils protéiques conduisant à l'identification bactérienne sans que la composition protéique de ces profils (*i.e.* la correspondance entre pic et protéine) soit connue (Welker and Moore 2011). Cependant, certains auteurs ont proposé d'utiliser cette méthodologie afin de détecter des biomarqueurs et/ou facteurs de virulence impliqués dans la pathogénie bactérienne (Bizzini and Greub 2010). Pour *S. aureus,* certains auteurs ont montré qu'il était possible de différencier les SARM des SASM. Cependant, la collection de souches utilisées était limitée (n=10 souches) et nécessitait l'utilisation de procédures de préparation de l'échantillon préalables (*i.e.* extractions chimiques et mécaniques) (Edwards-Jones, Claydon et al. 2000). D'autres auteurs ont affirmé qu'il était possible de détecter la PVL de *S. aureus* (Bittar, Ouchenane et al. 2009). Cependant, ces résultats se révélèrent non spécifiques et étaient probablement le reflet de l'appartenance à un même clone de la collection des souches analysées (Dauwalder, Carbonnelle et al. 2010; Szabados, Becker et al. 2011).

Dans le cadre de l'invention, les inventeurs ont ainsi démontré de façon totalement inattendue, que la présence sur le spectre de masse obtenu d'un pic à une valeur de m/z égale à 3005 ± 5 Th ou à 3035 ± 5 Th, et l'absence de pics à la fois à 3005 ± 5 Th et à 3035 ± 5 Th, pouvait être corrélée respectivement à la présence et à l'absence d'hémolysine δ ou de son variant, et donc permettait d'émettre un diagnostic clinique.

D'une manière préférée, dans le cadre du procédé selon l'invention, quelles que soient les variantes mises en oeuvre, la spectrométrie de masse est, avantageusement, réalisée sur un échantillon contenant une population de 10 à 10⁹ bactéries, et de préférence 10⁵ à 10⁶ bactéries. Le nombre de bactéries sera assimilé aux nombres d'Unités Formant Colonies (UFC) dénombrés si l'échantillon soumis à la spectrométrie de masse était déposé sur un milieu de culture gélosé standard pour la croissance de *Staphylococus aureus* après incubation pendant 18-48 heures à 35±2°C.

D'une manière générale, toute méthode de spectrométrie de masse par désorption-ionisation assistée par matrice et mesure du temps de vol peut être utilisée dans le cadre de l'invention.

Selon un mode de réalisation particulier, la spectrométrie de masse utilisée est la spectrométrie de masse (SM) [ou MS de l'anglais « *Mass Spectrometry* »] par désorption-ionisation assistée par matrice et mesure du temps de vol (MALDI-TOF) [MALDI-TOF de l'anglais « *Matrix Assisted Laser Desorption and Ionisation*/ *Time Of Flight* »], qui présente notamment l'avantage d'une mise en oeuvre relativement simple.

Selon un autre mode de réalisation particulier, la spectrométrie de masse utilisée est la SM en tandem MALDI-TOF-TOF, qui présente deux séparations successives en fonction du temps de vol et qui a notamment l'avantage de présenter une très bonne spécificité.

Préalablement à son ionisation, l'échantillon est préférentiellement mis en contact avec une matrice.

La matrice utilisée contient avantageusement un composé choisi parmi l'acide 3,5-diméthoxy-4-hydroxycinnamique (*i.e.* acide sinapique ou acide sinapinique) ; l'acide α-cyano-4-hydroxycinnamique (*i.e.* alpha-cyano, alpha-matrice ou CHCA), l'acide férulique et l'acide 2,5-dihydroxybenzoïque (*i.e.* DHB).

Il existe plusieurs techniques de dépôt qui pourront être utilisées dans le cadre de l'invention pour mettre l'échantillon en contact avec la matrice : dépôt sur une couche de matrice sèche, dit dépôt « couche mince », dépôt avec une goutte de matrice, dit dépôt « goutte séchée », dépôt sur une couche de matrice, puis rajout d'une goutte de matrice, dit dépôt « sandwich ».

Généralement, les matrices sont photosensibles et cristallisent en présence de l'échantillon tout en préservant l'intégrité des molécules. De telles matrices, notamment adaptées à la technique MS MALDI-TOF, sont bien connues et choisies parmi : l'acide 3,5-diméthoxy-4-hydroxycinnamique; l'acide α-cyano-4-hydroxycinnamique, l'acide férulique et l'acide 2,5-dihydroxybenzoïque. De nombreux autres composés sont connus de l'homme du métier. Il existe même des matrices liquides qui ne cristallisent ni à pression atmosphérique, ni même sous vide (Tholey and Heinzle 2006). Tout autre composé qui permettra d'ioniser les molécules de l'échantillon sous l'effet d'un rayon laser pourra être utilisé. Notamment, la cible, c'est-à-dire le support sur lequel est déposé l'échantillon, pourra directement jouer le rôle de matrice, comme dans le cas des techniques « *Nano-Assisted Laser Desorption*/*Ionization* » (NALDI) ou « Desorption/Ionization On silicon » (DIOS). Le rayon laser pourra avoir tout type de longueur d'onde favorable à la sublimation ou à la vaporisation de la matrice. De préférence, la longueur d'onde ultraviolette ou même infrarouge sera utilisée.

Dans la matrice, un tel composé est mis en solution, le plus souvent dans l'eau, de préférence de qualité « ultrapure », ou dans un mélange eau/solvant(s) organique(s). A titre d'exemple de solvants organiques classiquement utilisés, on peut citer, l'acétone, l'acétonitrile, le méthanol ou l'éthanol. L'acide trifluoroacétique (TFA) peut parfois être ajouté. Un exemple de matrice est, par exemple, constituée de 20 mg/mL d'acide sinapique dans un mélange acétonitrile/eau/TFA de 50/50/0,1 (v/v). Le solvant organique permet aux molécules hydrophobes présentes dans l'échantillon de se dissoudre dans la solution, alors que l'eau permet la dissolution des molécules hydrophiles. La présence d'acide, tel que le TFA, favorise l'ionisation des molécules de l'échantillon par captation d'un proton (H⁺).

Préférentiellement, l'échantillon est déposé sur un support nommé cible, le plus souvent sous la forme de spots. La matrice peut être directement déposée sur l'échantillon et se mélange alors à ce dernier.

De façon optionnelle, le procédé selon l'invention contient en outre avant l'étape b), une étape de cristallisation de la matrice sur ou dans laquelle les molécules de l'échantillon sont adsorbées.

Le plus souvent, la cristallisation de la matrice est obtenue en laissant sécher la matrice dans ou sur laquelle l'échantillon a été déposé.

Selon un mode de réalisation particulier, le milieu avec lequel l'échantillon est mis en contact correspond à une matrice adaptée à la SM MALDI déposée sur une cible, et le procédé contient avant l'étape b), l'obtention de la cristallisation de la matrice sur ou dans laquelle les molécules de l'échantillon sont adsorbées. Dans ce cas, la matrice contient préférentiellement un composé choisi parmi l'acide 3,5-diméthoxy-4-hydroxycinnamique, l'acide α-cyano-4-hydroxycinnamique, l'acide férulique et l'acide 2,5-dihydroxybenzoïque.

L'échantillon peut au préalable avoir été mis en culture dans un bouillon ou sur une gélose de manière à l'enrichir en bactéries de *Staphylococcus aureus*. De tels milieux, gélosés ou en bouillon, sont bien connus de l'homme de l'art. On peut, par exemple, citer la gélose au sang (de cheval ou de mouton) ; la gélose columbia ; la gélose « chocolat » polyvitex^{®} ; la gélose chromogénique ChromID Staph^{®} pour la recherche de *S. aureus* de la société bioMérieux, *etc.* L'enrichissement sur gélose est particulièrement favorable puisqu'il permet d'obtenir des colonies pures de *S. aureus* qui pourront être déposées sur la cible. Le solvant présent dans la matrice est ensuite vaporisé, par exemple, en laissant l'échantillon à une température appartenant, par exemple, à la gamme allant de 17 à 30°C, et notamment à température ambiante (22°C) pendant quelques minutes, par exemple de 5 minutes à 2 heures. Cette vaporisation du solvant permet la cristallisation de la matrice dans laquelle l'échantillon est réparti. Ensuite, l'échantillon, placé au sein de la matrice cristallisée, est soumis à une ionisation douce. Cette ionisation sera, de préférence, réalisée avec un laser à azote émettant un rayon UV à 337.1 nm.

Lors de l'ionisation, l'échantillon est soumis à une excitation par laser. Les cristaux de la matrice absorbent alors l'énergie photonique et la restitution de cette énergie entraîne la sublimation de la matrice, la désorption de l'échantillon et l'apparition de matière dans un état qualifié de plasma. Au sein de ce plasma, il se produit des échanges de charges entre molécules de matrice et d'échantillon. Par exemple, des protons peuvent être arrachés à la matrice et transférés aux protéines et aux peptides de l'échantillon. Cette étape permet une ionisation douce des biomolécules sans induire leurs destructions. Les échantillons libèrent ainsi des ions de différentes tailles. Ces derniers sont alors accélérés par un champ électrique et volent librement dans un tube sous pression réduite, nommé tube de vol. La pression appliquée lors de l'ionisation et lors de l'accélération des ions générés appartient le plus souvent à la gamme allant de 10⁻⁶ à 10⁻⁹ millibar [mbar]. Les ions les plus petits vont alors « voyager » plus rapidement que les ions plus gros permettant ainsi leurs séparations. A l'extrémité terminale du tube de vol est situé un détecteur. Le temps de vol (ou TOF pour « *Time Of Flight* » en anglais) mis par les ions est utilisé pour calculer leur massé. Ainsi, un spectre de masse est obtenu, représentant l'intensité du signal correspondant au nombre de molécules ionisées d'une même masse sur charge [m/z], en fonction du rapport m/z des molécules qui frappent le détecteur. Le rapport masse sur charge [m/z] est exprimé en Thomson [Th]. Une fois introduite dans le spectromètre de masse, le spectre d'un échantillon est obtenu très rapidement, le plus souvent en moins d'une minute.

Le fait que l'échantillon analysé contienne bien des bactéries de *Staphylococcus aureus* peut être déterminé par un test préalable à la MS MALDI-TOF ou de manière concomitante grâce au spectre de MS MALDI-TOF. Dans ce cas, le spectre de MS MALDI TOF total, qui comprend le plus souvent entre 70 et 200 pics, sera comparé à une base de données spectrale qui permet de déterminer si la population bactérienne analysée correspond bien à *Staphylococcus aureus.* Cette comparaison repose sur l'utilisation de différents algorithmes selon les fournisseurs, et conduit à l'obtention de l'identification bactérienne (Cherkaoui, Hibbs et al. 2010; Welker and Moore 2011).

Tous les modes de réalisation détaillés ci-dessus sont applicables quelle que soit la technique de spectrométrie de masse utilisée, en particulier de type MALDI-TOF ou MALDI-TOF-TOF.

Lorsque la spectrométrie de masse utilisée est la SM MALDI-TOF, la détermination de l'étape g) est de préférence directement déduite de la présence sur le spectre de masse obtenu d'un pic à une valeur de m/z égale à 3005 ± 5 Th ou à 3035 ± 5 Th ou de l'absence de pics à la fois à 3005 ± 5 Th et à 3035 ± 5 Th.

Lorsque la spectrométrie de masse utilisée est la SM MALDI-TOF-TOF, on laisse de préférence se déplacer les molécules ionisées dans un premier tube sous pression réduite à l'étape d), on sélectionne les ions de masse m/z égale à 3005 ± 5 Th et/ou de masse m/z égale à 3035 ± 5 Th, on accélère à nouveau les molécules ionisées après leur fragmentation, par exemple par collision, et on laisse se déplacer librement dans un deuxième tube les molécules ionisées fragmentées ou non.

Avec la SM MALDI-TOF-TOF, la détermination de l'étape g) est de préférence indirectement déduite de la présence d'au moins cinq fragments, et de préférence d'au moins 10 fragments, parmi les ions fragments y et b suivants :
- les ions fragments y de masse 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2005.148, 2118.232, 2219.280, 2306.312, 2419.396, 2532.480, 2647.507, 2775.565, 2846.603 et 2976.635, avec une tolérance de ± 1.5 Th ; et les ions fragments b de masse 131.040, 202.077, 330.136, 445.163, 558.247, 671.331, 758.363, 859.410, 972.494, 1029.516, 1144.543, 1257.627, 1356.695, 1484.790, 1670.870, 1783.954, 1897.038, 2012.065, 2113.112, 2212.181, 2326.224, 2454.319, 2601.387, 2702.435, 2830.530 et 2958.625, avec une tolérance de ± 1.5 Th, pour le pic à 3005 ± 5 Th, et/ou
- les ions fragments y de masse 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2035.159, 2148.243, 2249.290, 2336.322, 2449.406, 2562.491, 2677.517, 2805.576, 2876.613 et 3006.646, avec une tolérance de ± 1.5 Th ; et les ions fragments b de masse 131.040, 202.077, 330.136, 445.163, 558.247, 671.331, 758.363, 859.410, 972.494, 1059.526, 1174.553, 1287.638, 1386.706, 1514.801, 1700.880, 1813.964, 1927.048, 2042.075, 2143.123, 2242.191, 2356.234, 2484.329, 2631.398, 2732.445, 2860.540 et 2988.635, avec une tolérance de ± 1.5 Th, pour le pic à 3035 ± 5 Th.

Un procédé de spectrométrie de masse MALDI-TOF utilisable selon l'invention peut notamment comprendre les étapes successives suivantes pour l'obtention du spectre de masse :
- Placer l'échantillon à étudier à la surface ou dans une matrice adaptée à la spectrométrie de masse par désorption-ionisation assistée sur matrice par temps de vol,
- Obtenir la cristallisation de la matrice sur ou dans laquelle les molécules de l'échantillon sont adsorbées,
- Ioniser le mélange échantillon/matrice cristallisée, grâce à un rayon laser,
- Accélérer les molécules ionisées obtenues grâce à une différence de potentiel,
- Laisser se déplacer librement les molécules ionisées et accélérées dans un tube sous pression réduite,
- Détecter les molécules ionisées en sortie du tube, de manière à mesurer le temps qu'elles ont mis pour parcourir le tube sous pression réduite et à obtenir un signal correspondant au nombre de molécules ionisées atteignant le détecteur à un instant donné,
- Calculer le rapport masse sur charge [m/z] des molécules détectées, de manière à obtenir un signal correspondant au nombre de molécules ionisées d'une même masse sur charge [m/z], en fonction du rapport m/z des molécules détectées.

En général, le calcul du rapport m/z est obtenu, en tenant compte de la calibration préalable du spectromètre de masse utilisé, sous la forme d'une équation liant le rapport masse sur charge [m/z] et le temps de parcours dans le tube sous pression réduite des molécules ionisées.

La MS MALDI-TOF qui peut être utilisée dans le cadre de l'invention repose sur la mesure du temps de parcours d'un point A à un point B d'ions de différentes charges et de différentes masses exposés à un champ électrique. La mesure de ce temps de parcours est dépendante de la masse et de la charge de l'ion, permettant ainsi sa séparation (Welker and Moore 2011).

Un procédé MALDI-TOF-TOF qui peut également être utilisé dans le cadre de l'invention peut notamment comprendre les étapes suivantes pour l'obtention du spectre de masse :
- Placer l'échantillon à étudier à la surface ou dans une matrice adaptée à la MS par MALDI-TOF-TOF,
- Obtenir la cristallisation de la matrice sur ou dans laquelle les molécules de l'échantillon sont adsorbées,
- Ioniser le mélange échantillon/matrice cristallisée, grâce à un rayon laser,
- Accélérer les molécules ionisées obtenues grâce à une différence de potentiel,
- Laisser se déplacer librement les molécules ionisées et accélérées dans un premier tube sous pression réduite,
- A l'issue de la traversée du premier tube, sélectionner les molécules ionisées ayant la masse de l'ion moléculaire à fragmenter et éliminer tous les autres ions, par exemple à l'aide d'un déflecteur électrostatique,
- Obtenir la fragmentation des molécules ionisées, de façon optionnelle en la provoquant à l'aide d'un gaz inerte,
- Augmenter à nouveau le potentiel pour provoquer l'accélération des molécules ionisées, par exemple à l'aide de grilles de potentiel de façon à conférer une énergie cinétique différente aux ions fragments et à l'ion précurseur,
- Laisser se déplacer librement les molécules ionisées fragmentées ou non dans un deuxième tube sous pression réduite,
- Détecter les molécules ionisées en sortie du tube, de manière à mesurer le temps qu'elles ont mis pour parcourir le tube sous pression réduite et à obtenir un signal correspondant au nombre de molécules ionisées atteignant le détecteur à un instant donné,
- Calculer le rapport masse sur charge [m/z] des molécules détectées, de manière à obtenir un signal correspondant au nombre de molécules ionisées d'une même masse sur charge [m/z], en fonction du rapport m/z des molécules détectées.

En général, le calcul du rapport m/z est obtenu, en tenant compte de la calibration préalable du spectromètre de masse utilisé, sous la forme d'une équation liant le rapport masse sur charge [m/z] et le temps de parcours dans le tube sous pression réduite des molécules ionisées. La masse de l'ion précurseur peut servir à réaliser cette calibration.

Tout type de spectromètre de masse MALDI-TOF ou MALDI-TOF/TOF peut être utilisé pour l'élaboration du spectre de masse. De tels spectromètres comprennent :
*i)* une source d'ionisation (en général un laser UV) destinée à ioniser le mélange échantillon/matrice ;
*ii)* un accélérateur des molécules ionisées par application d'une différence de potentiel,
*iii)* un tube sous pression réduite dans lequel se déplacent les molécules ionisées et accélérées,
*iv)* un analyseur de masse destiné à séparer les ions moléculaires formés, en fonction de leur ratio masse sur charge (m /z) ;
*v)* un détecteur destiné à mesurer le signal produit directement par les ions moléculaires.

Un spectromètre de masse MALDI-TOF-TOF est identique à un spectromètre de masse MALDI-TOF, à la différence près que le tube sous pression réduite est divisé en deux parties situées dans le prolongement l'une de l'autre (également nommées ici premier tube et deuxième tube sous pression réduite). Cette géométrie permet deux séparations successives des ions moléculaires en fonction du temps de vol : la première permet la sélection d'un ion moléculaire qui sera séparé en fragments dans la deuxième partie du tube. On entend par fragments toute structure moléculaire obtenue en cassant l'ion moléculaire sélectionné.

La fragmentation des ions peut être obtenue soit durant l'ionisation initiale, soit dans une cellule de collision située entre les deux parties du tube ci-dessus mentionnées. Lors de l'ionisation initiale, la fragmentation peut être directement provoquée par l'énergie du rayon laser absorbée par les molécules ou être provoquée par la collision entre molécules, neutres ou ionisées dans le plasma moléculaire. Si la fragmentation intervient avant la phase d'accélération des ions, les fragments migrent selon leurs masses respectives. Si elle intervient pendant la phase d'accélération, la séparation des ions, dits métastables, est médiocre. Si elle intervient après l'accélération initiale, les ions fragments et leur molécule mère ionisée (ion précurseur) ont la même énergie cinétique et migrent à la même vitesse. Ce dernier type de fragmentation, baptisée fragmentation post-source, est mis à profit dans la technique MS MALDI-TOF-TOF. La première partie du tube (ou encore nommée ici premier tube sous pression réduite) permet de sélectionner une fenêtre de masses incluant la masse de l'ion précurseur d'intérêt et bornée par un delta de masse. L'ion précurseur et ses fragments sont ensuite à nouveau accélérés entre les deux parties du tube à pression réduite. Les ions acquièrent alors une énergie cinétique qui est fonction de leur masse. Ils migrent finalement dans la deuxième partie du tube sous pression réduite (ou encore nommée ici deuxième tube sous pression réduite) en fonction de leurs masses respectives. Il est ainsi possible de détecter l'ensemble des fragments générés pour un ion précurseur donné.

Une variante de cette méthode consiste à ajouter dans la cellule de collision un gaz inerte, tel que de l'argon, entre les deux parties du tube sous pression réduite. Les molécules ionisées se fragmentent alors en rentrant en collision avec le gaz inerte. Elles peuvent ensuite être accélérées et séparées comme précédemment.

Une SM par MALDI TOF/TOF fragmente les protéines de façon privilégiée autour de la liaison peptidique, il génère essentiellement des ions de type b et de type y selon la nomenclature usuelle (Paizs and Suhai 2005). D'autres fragments sont également possibles, ions immonium, fragment a, *etc.*

L'homme de l'art a l'habitude d'utiliser le profil de fragmentation pour générer des informations sur la séquence en acides aminés de la protéine et pour essayer d'identifier la séquence protéique. Il utilise notamment à cette fin des logiciels tels que Mascot de la société Matrix Science (Londres, Royaume-Uni).

Ainsi l'hémolysine δ de SEQ ID N°1 conduira majoritairement :
- aux ions fragments y de masse 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2005.148, 2118.232, 2219.280, 2306.312, 2419.396, 2532.480, 2647.507, 2775.565, 2846.603 et 2976.635;
- et aux ions fragments b de masse 131.040, 202.077, 330.136, 445.163, 558.247, 671.331, 758.363, 859.410, 972.494, 1029.516, 1144.543, 1257.627, 1356.695, 1484.790, 1670.870, 1783.954, 1897.038, 2012.065, 2113.112, 2212.181, 2326.224, 2454.319, 2601.387, 2702.435, 2830.530 et 2958.625.

De même, l'hémolysine δ G10S de SEQ ID N°2 conduira majoritairement :
- aux ions fragments y de masse 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2035.159, 2148.243, 2249.290, 2336.322, 2449.406, 2562.491, 2677.517, 2805.576, 2876.613 et 3006.646 ;
- et aux ions fragments b de masse 131.040, 202.077, 330.136, 445.163, 558.247, 671.331, 758.363, 859.410, 972.494, 1059.526, 1174.553, 1287.638, 1386.706, 1514.801, 1700.880, 1813.964, 1927.048, 2042.075, 2143.123, 2242.191, 2356.234, 2484.329, 2631.398, 2732.445, 2860.540 et 2988.635.

Les exemples, en relation avec les Figures annexées, ci-après permettent d'illustrer l'invention mais n'ont aucun caractère limitatif.
La **Figure 1** représente la séquence primaire de l'hémolysine δ (Fitton, Dell et al. 1980).
La **Figure 2** met en évidence la détection de l'hémolysine δ par MS MALDI TOF à partir de souches isogéniques de *Staphylococcus aureus.*
La **Figure 3** étudie l'effet du temps de culture (18 à 48h) sur la détection de l'hémolysine δ par MS MALDI TOF de 3 souches isogéniques et de 2 souches cliniques.
La **Figure 4** illustre la détection de l'hémolysine δ et de son variant G10S par MS MALDI TOF à partir de souches cliniques de *Staphylococcus aureus.*

### EXEMPLES

### 1.Matériel

### •Matrice

Utilisation d'une matrice d'acide α-cyano-4-hydroxy cinnamique [CHCA] prête à l'emploi (Référence 411071, bioMérieux, Marcy l'Etoile, France).

### •Cibles et contrôle interne

Utilisation de cibles jetables Vitek MS DS^{®} (Référence 410893 bioMérieux, Marcy l'Etoile, France). Utilisation d'une souche *d'Escherichia coli* ATCC 8739 à chaque analyse permettant de calibrer ou de vérifier la validité de la calibration du spectromètre de masse.

### •Type de dépôts

Les souches de *Staphylococcus aureus* sont déposées sous forme d'un étalement fin d'une pointe de colonie (étalement sous forme de couche fine ou « *smear* » en anglais). Cet étalement est séché pendant 5 à 15 minutes à température ambiante. Un microlitre de matrice CHCA est alors déposé puis séché pendant 5 à 15 minutes environ à température ambiante.

### Spectromètre de masse MALDI TOF

Utilisation d'un spectromètre de masse de type MALDI TOF modèle Axima Assurance^{®} de la société Shimadzu, Champs sur Marne, France.

### •Paramétrage utilisé lors de l'analyse sur spectromètre de masse MALDI TOF

Lors des analyses des souches de *S. aureus,* les paramètres de réglages suivants du spectromètre de masse ont été utilisés :
- Zone de détection des masses : 2000 - 20 000 Th
- Puissance du laser : 80 volts
- Fréquence du laser : 50 hertz
- Type de laser : N2
- Mode du spectromètre de masse : linéaire, positif
- Puissance d'extraction : 8330 Th
- Mode « auto qualité » activé
- Intensité minimale : 10 mV
- Rapport signal sur bruit minimal : 10
- Résolution minimale acceptable : 300
- Nombre de tirs : 5 tirs / profils
- Nombre de profils par spectre : 100

### •Logiciels

Les plans de plaque sont réalisés à l'aide du logiciel Sirweb-Maldi-Tof^{®} version 11 (I2A, Peyrols, France) ou du logiciel Target Manager^{®} version 1.12 (bioMérieux, Marcy l'Etoile, France). Le spectromètre de masse est piloté par le logiciel LaunchPad^{®} version 2.8.4.20081127 (Shimadzu, Champs sur Marne, France).

### 2. Méthodologie

### •Paramètres pré analytiques

Analyse des souches après subculture de 18 à 24h sur gélose au sang de cheval (TSH, Référence 43061, bioMérieux, Marcy l'Etoile, France) à 35 ± 2°C sous atmosphère aérobie.

Calibration initiale additionnelle (à celle utilisée pour l'identification bactérienne) du spectromètre de masse dans la zone 2800 à 3200 m/z à l'aide d'un calibrant pour spectrométrie de masse composé de polyéthylène glycol (PEG) 3000.

### •Paramètres analytiques

A partir d'une pointe de colonie, réalisation du dépôt sur une cible jetable selon la « technique du *smear* » utilisée en routine lors de l'identification des bactéries.

Séchage 5-15 minutes à température ambiante.

Ajout de 1µL de matrice CHCA sur le précédent dépôt.

Séchage pendant 5-15 minutes.

Réalisation du « plan de plaque » à l'aide du logiciel Sirweb-Maldi-Tof^{®} ou de Target Manager^{®}.

Démarrage de l'analyse par le MS MALDI TOF à l'aide du logiciel Launchpad^{®} de contrôle du spectromètre de masse Axima Assurance^{®}.

### 3. Résultats

### a. Validation de la détection

### i. Utilisation de souches isogéniques de Staphylococcus aureus

L'analyse de souches isogéniques pour le système *agr*/*rnaIII*/*hld* obtenues par remplacement allélique du locus agr a permis de démontrer la présence d'un pic à 3005 ± 5 Th pour les souches présentant un système agr fonctionnel. A l'inverse, les souches isogéniques, identiques aux souches parentales précédentes à l'exception d'un système agr muté, ne présentaient pas de pic à 3005 ± 5 Th. La souche RN6390 testée possède un système agr fonctionnel et est productrice d'hémolysine δ ; la souche LUG950 est issue de la souche RN6390 dans laquelle le gène codant l'ARN III a été invalidé : la souche est déficiente pour la synthèse d'hémolysine δ ; la souche RN6911 est une souche obtenue par délétion complète du locus agr (ARN II et ARN III) de la souche RN6390 : la souche est déficiente pour la synthèse d'hémolysine δ. Le **Tableau I** et la **Figure 2** résument les résultats obtenus. La **Figure 2** présente le spectre de masse **WC-MS MALDI TOF** obtenu avec les trois souches testées pour des rapports m/z compris entre 2800 et 3200.

**Tableau I : Détection de l'hémolysine δ par WC-MS MALDI TOF et par recherche de l'hémolyse en milieu gélosé sur une collection de souches isogéniques**

| Référence souche | Caractéristiques | Test de synergie d'hémolyse sur gélose au sang | Présence d'un pic à 3005 ± 5 Th |
|---|---|---|---|
| RN6390 | Souche parentale *agr*⁺ | + | + |
| LUG 950 | RN6390 Δ*rnaIII* | - | - |
| RN 6911 | RN6390 Δ*agr* | - | - |

### ii. Données de répétabilité et de fidélité intermédiaire

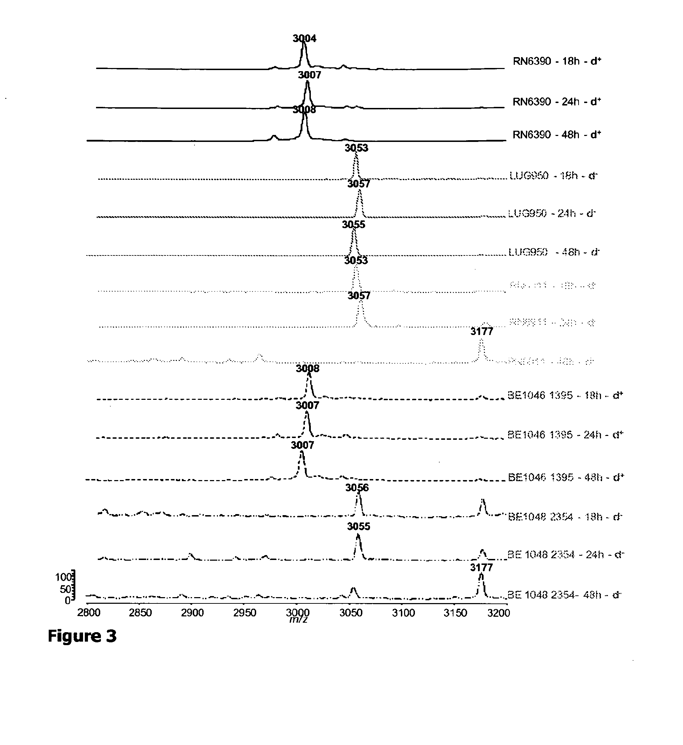

Afin de pouvoir s'affranchir de conditions culturales trop strictes nécessaires à une utilisation de cette détection en pratique quotidienne, des données de répétabilité et de fidélité intermédiaire ont été établies. Dans ce but, ont été utilisées les 3 souches isogéniques précédemment décrites (*i.e.* RN6390 ; LUG 950 ; et RN 6911) et 2 souches cliniques, l'une exprimant l'hémolysine δ (*i.e.* BE1046 1395), l'autre pas (*i.e.* BE1048 1354). Ces souches ont été cultivées pendant 18, 24 et 48 heures sur des géloses au sang de cheval (TSH, Référence 43061, bioMérieux, Marcy l'Etoile, France). Ces souches ont été alors testées en dupliquât en WC-MS MALDI TOF. Dans tous les cas, et à tous les temps de cultures, le pic à 3005 ± 5 Th a été détecté chez toutes les souches productrices d'hémolysine δ et s'est montré absent des souches présentant un dysfonctionnement du système agr **(****Figure 3****).**

### iii. Influence des milieux de culture sur la détection de l'hémolysine δ

L'influence des milieux de culture a également été évaluée. Les 5 mêmes souches (les 3 souches isogéniques et les 2 souches cliniques, une exprimant l'hémolysine δ, l'autre pas), ont été cultivées sur 5 milieux différents pendant 24h à 35 ± 2°C sous aérobie : gélose au sang de cheval (TSH, Référence 43061, bioMérieux, Marcy l'Etoile, France) ; gélose columbia (COS, Référence 43041, bioMérieux, Marcy l'Etoile, France) ; gélose « chocolat » (PVX, Référence 43101, bioMérieux, Marcy l'Etoile, France) ; gélose P (GP, Bacto-Peptone DIFCO^{®}, Becton Dickinson, Pont de Claix, France) ; et gélose chromogénique pour la recherche de *S. aureus* (ChromID Staph^{®}, Référence 43371, bioMérieux, Marcy l'Etoile, France). Ces différentes conditions culturales n'ont pas d'influence sur la détection de l'hémolysine δ par WC-MS MALDI TOF à l'aide du pic à 3005 ± 5 Th comme cela apparait sur le **Tableau II.**

**Tableau II : Influence des conditions de culture sur la détection de l'hémolysine δ.**

| Souche | TSH^{®} | COS^{®} | PVX^{®} | GP | ChromID Staph^{®} |
|---|---|---|---|---|---|
| RN6390 | + | + | + | + | + |
| LUG 950 | - | - | - | - | - |
| RN 6911 | - | - | - | - | - |
| BE10482354 | - | - | - | - | - |
| BE10461395 | + | + | + | + | + |

| | | | | | |
|---|---|---|---|---|---|
| + : pic à 3005 ± 5 Th détecté. - : absence de pic à 3005 ± 5 Th. | | | | | |

### iv. Analyse prospective d'une collection de souches cliniques provenant du laboratoire de microbiologie du Centre de Biologie et Pathologie Est des Hospices Civils de Lyon.

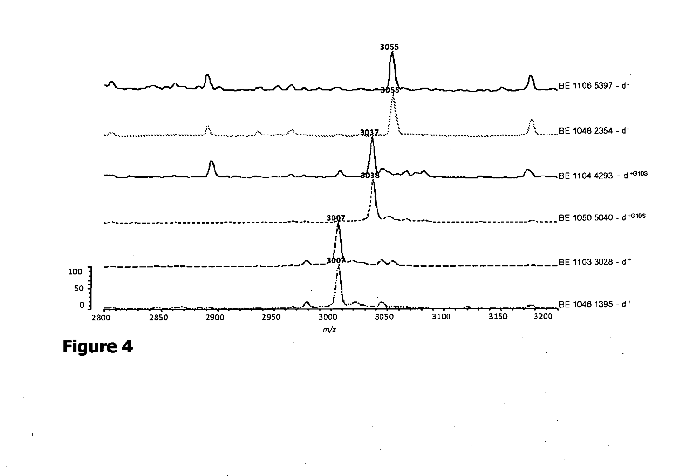

Afin d'étudier l'impact clinique du dysfonctionnement du système agr, objectivé par l'absence du pic 3005 ± 5 Th, une collection de 168 isolats a été collectée prospectivement entre novembre 2010 et mars 2011 au sein du laboratoire de bactériologie du Centre de Biologie et Pathologie Est [CBPE] des Hospices Civils de Lyon [HCL]. Parmi eux, 139 (82,7%) présentaient, en WC-MS MALDI TOF un pic à 3005 ± 5 Th ; 12 (7,2 %) possédaient un pic à 3035 ± 5 Th et non à 3005 ± 5 Th ; et enfin 17 souches (10,1%) ne montraient ni pic à 3005 ± 5 Th, ni pic à 3035 ± 5 Th. Afin de confirmer les résultats obtenus par une seconde méthodologie, des tests d'hémolyse sur gélose au sang ont été effectués. Les 17 souches ne présentant aucun pic à 3005 ± 5 Th et à 3035 ± 5 Th ne montrèrent aucune hémolyse. A l'inverse, les 10 souches dénuées de pics à 3005 ± 5 Th mais possédant un pic à 3035 ± 5 Th présentèrent un test d'hémolyse positif. Enfin, la détection de l'hémolysine δ par le test d'hémolyse sur 5 des 139 souches prises au hasard et présentant un pic à 3005 ± 5 Th se révéla positive. Afin d'expliquer la présence d'une hémolyse associée à un pic à 3035 ± 5 Th sans pic à 3005 ± 5 Th, le gène *hld* codant l'hémolysine δ a été séquencé. Pour les 10 souches, une mutation entraînant la substitution d'une glycine par une sérine en position 10 (*i.e.* G10S) a été détectée, ce qui explique la modification de masse mesurée par MS MALDI TOF **(****Figure 4****).** Parmi les souches dont le spectre WC-MS MALDI TOF pour un rapport m/z compris entre 2800 et 3200 est représenté **Figure 4****,** les souches BE1046 1395 et BE1103 3028 produisent de l'hémolysine δ sous forme sauvage (δ⁺); les souches BE1050 5040 et BE1104 4293 produisent de l'hémolysine δ présentant la mutation G10S (δ^{+G10S}); les souches BE1106 5397 et BE1048 2354 sont déficientes en hémolysine δ (δ⁻).

### v. Analyse MALDI TOF/TOF

Une analyse par spectrométrie de masse « *MALDI-TOF*/*Time Of Flight* » [MALDI TOF-TOF] sur 9 souches a été réalisée : les 3 souches isogéniques précédemment décrites (*i.e.* RN6390 ; LUG 950 ; et RN 6911), 2 souches cliniques non hémolytiques ne présentant ni pic à 3005 ± 5 Th, ni pic à 3035 ± 5 Th, 2 souches cliniques hémolytiques montrant un pic à 3005 ± 5 Th ; et deux souches cliniques hémolytiques présentant un pic à 3035 ± 5 Th mais dénué de pic à 3005 ± 5 Th.

Ainsi, l'analyse en mode MALDI-TOF-TOF du pic à 3005 ± 5 Th de la souche RN6390 a permis de détecter les fragments de l'hémolysine δ de masse 147.252, 201.359, 275.471, 330.459, 376.514, 446.482, 523.633, 559.574, 651.7, 672.606, 757.71, 765.841, 864.832, 965.922, 1080.95, 1194.071, 1307.149, 1356.133, 1493.23, 1621.321, 1672.21, 1720.294, 1785.044, 1833.275, 1948.428, 2005.551, 2119.576, 2219.583, 2307.544, 2420.56, 2533.786, 2600.009, 2602.657, 2647.743, 2774.373, 2776.851 et 2975.308 Th. Ces fragments correspondent respectivement aux ions y1, y2, y3, y4, y5, y6, y7, y8, y9, y10, y11, y12, y13, y14, y15, y16, y17, y18, y19, y20, y21, y22, y23, y24, y26, b2, b3, b4, b5, b6, b7, b13, b15, b16 et b23 de l'hémolysine δ de séquence **SEQ ID N°1** avec une tolérance de ± 1.5 Th. Plus de 5 fragments appartenant à l'hémolysine δ de séquence **SEQ ID N°1** ont ainsi été détectés.

De même, l'analyse en mode MALDI-TOF-TOF du pic à 3035 ± 5 Th de la souche BE1050 5040 a permis de détecter les fragments de l'hémolysine δ G10S de masse 275.408, 330.363, 376.414, 446.463, 523.39, 559.462, 651.572, 757.505, 765.682, 860.673, 864.661, 965.714, 1080.77, 1175.788, 1193.829, 1306.922, 1493.064, 1621.166, 1701.34, 1720.251, 1814.587, 1833.381, 1948.485, 2035.545, 2148.557, 2249.99, 2336.585, 2449.681, 2562.833, 2677.454 et 2677.935 Th. Ces fragments correspondent respectivement aux ions y2, y3, y4, y5, y6, y7, y8, y9, y10, y11, y12, y13, y14, y15, y16, y17, y18, y19, y20, y21, y22, y23, b3, b4, b5, b7, b8, b11, b15 et b16 de l'hémolysine δ G10S de séquence **SEQ ID N°2** avec une tolérance de ± 1.5 Th. Plus de 5 fragments appartenant à l'hémolysine δ de séquence **SEQ ID N°2** ont ainsi été détectés.

A l'inverse l'analyse MALDI-TOF-TOF du pic à 3053 ± 5 Th de la souche LUG 950 n'a pas permis de détecter les fragments de masse correspondant aux **SEQ ID N°1** ou **SEQ ID N°2.** Seuls les pics de masse suivants ont été détectés : 72.826, 96.485, 128.727, 214.235, 242.298, 263.249, 267.247, 285.276, 362.359, 476.458, 1206.984 et 1848.786 Th.

Dans tous les cas, l'analyse par spectrométrie de masse MALDI TOF-TOF et la recherche dans la base de données Mascott^{®} (Matrix Science Ltd, London, UK) a confirmé la totalité des résultats précédemment obtenus, notamment la correspondance du pic à 3035 ± 5 Th avec la forme mutée d'hémolysine δ G10S.

Ces exemples permettent de montrer que le mode MALDI-TOF, bien que plus simple à mettre en oeuvre, conduit aux mêmes identifications que le mode MALDI-TOF-TOF qui est plus spécifique.

La totalité des résultats obtenus est résumée dans le **Tableau III.**

**Tableau III : Détection de l'hémolysine δ par spectrométrie de masse MALDI TOF dans 168 souches cliniques et corrélation avec le test de synergie d'hémolyse en diffusion et la détection par spectrométrie MALDI TOF/TOF.**

| | Pic à 3005 ± 5 Th (Nombre de souches positives /nombre de souches analysées) | Pic à 3035 ± 5 Th (Nombre de souches positives /nombre de souches analysées) | Absence de pics à 3005 ± 5 Th et 3035 ± 5 Th (Nombre de souches positives /nombre de souches analysées) |
|---|---|---|---|
| MALDI TOF | 141/168 | 10/168 | 17/168 |
| Test de synergie d'hémolyse sur gélose au sang | 5/5 | 10/10 | 0/17 |
| MALDI TOF/TOF | 2/2 | 2/2 | 0/2 |

Enfin, afin de se prémunir de tous biais dus à l'inclusion de souches appartenant à un même clone, les 17 souches déficientes en hémolysine δ ont été analysées par puces à gènes (IDENTIBAC^{®}, Alere, France), montrant leur appartenance à 4 complexes clonaux et à 3 fonds génétiques différents.

### vi. Lien avec la chronicité de l'infection.

En parallèle au recueil des 168 isolats, des informations cliniques ayant trait au caractère aigue *versus* chronique de l'infection ou de la colonisation à *S*. *aureus* ont été collectées. Une infection chronique est définie sur la base du critère clinico-biologique consistant à retrouver l'isolement, jusqu'à 6 mois auparavant, d'une souche de *S. aureus* présentant le même antibiogramme que la souche isolée lors de l'étude. Dans tous les autres cas, l'infection ou la colonisation étaient catégorisées comme aigue. Par ailleurs, la présence de matériels implantables : cathéters, prothèses ostéo-articulaires ou vasculaires ; fréquemment retrouvée comme associée aux infections chroniques (Hawkins, Huang et al. 2007), a également été recueillie.

Sur 34 souches isolées au cours d'infections chroniques, 11 ne produisaient pas d'hémolysine δ contre 6 sur 134 isolées au cours d'infections aigues, objectivant un lien statistiquement significatif entre déficience en hémolysine δ et infections chroniques en analyse univariée (*p*=0.001). De même, l'analyse statistique multivariée retrouve ce lien entre défaut en hémolysine δ et chronicité de l'infection, tant pour les SASM (*p*=0.023) que pour les SARM (*p*=0.082). A l'inverse, aucun lien entre l'absence de détection d'hémolysine et la présence de matériels implantables n'a pu être retrouvé (*p*=0.470).

### vii. Lien avec GISA

La recherche de GISA a été effectuée par méthodes phénotypiques sur les 168 souches collectées prospectivement. Cinq se sont révélées GISA et 4 de ces 5 souches ne présentaient aucun pic à 3005 ± 5 Th et à 3035 ±₋ 5 Th (δ⁻). Compte tenu du faible effectif de souches GISA obtenues dans cette étude, une collection de 28 souches de GISA parfaitement caractérisées par le Centre National de Référence des Staphylocoques a été analysée vis-à-vis de leur production d'hémolysine δ. Neuf souches sur 28 étaient δ⁻ contre 13/163 souches non GISA issues de l'étude clinique, confirmant, là encore, le lien entre dysfonction du système agr et le phénotype de résistance GISA (*p=*0,001).

### Références

Balaban, N. and R. P. Novick (1995). "Translation of RNAIII, the Staphylococcus aureus agr regulatory RNA molecule, can be activated by a 3'-end deletion." FEMS Microbiol Lett 133(1-2): 155-161.
Bittar, F., Z. Ouchenane, et al. (2009). "MALDI-TOF-MS for rapid detection of staphylococcal Panton-Valentine leukocidin." Int J Antimicrob Agents 34(5): 467-470.
Bizzini, A. and G. Greub (2010). "Matrix-assisted laser desorption ionization time-of-flight mass spectrometry, a revolution in clinical microbial identification." Clin Microbiol Infect 16(11): 1614-1619.
Boles, B. R. and A. R. Horswill (2008). "Agr-mediated dispersal of Staphylococcus aureus biofilms." PLoS Pathog 4(4): e1000052.
Brunskill, E. W. and K. W. Bayles (1996). "Identification and molecular characterization of a putative regulatory locus that affects autolysis in Staphylococcus aureus." J Bacteriol 178(3): 611-618.
Cherkaoui, A., J. Hibbs, et al. (2010). "Comparison of two matrix-assisted laser desorption ionization-time of flight mass spectrometry methods with conventional phenotypic identification for routine identification of bacteria to the species level." J Clin Microbiol 48(4): 1169-1175.
Dauwalder, O., E. Carbonnelle, et al. (2010). "Detection of Panton-Valentine toxin in Staphylococcus aureus by mass spectrometry directly from colony: time has not yet come." Int J Antimicrob Agents 36(2): 193-194.
Dufour, P., S. Jarraud, et al. (2002). "High genetic variability of the agr locus in Staphylococcus species." J Bacteriol 184(4): 1180-1186.
Edwards-Jones, V., M. A. Claydon, et al. (2000). "Rapid discrimination between methicillin-sensitive and methicillin-resistant Staphylococcus aureus by intact cell mass spectrometry." J Med Microbiol 49(3): 295-300.
Felden, B., F. Vandenesch, et al. (2011). 'The Staphylococcus aureus RNome and its commitment to virulence." PLoS Pathog 7(3): e1002006.
Figueiredo, A. M., S. Jarraud, et al. (2000). "Direct Submission." 2000, from http://www.ncbi.nlm.nih.gov/protein/AAG03054.1.
Fitton, J. E., A. Dell, et al. (1980). "The amino acid sequence of the delta haemolysin of Staphylococcus aureus." FEBS Lett 115(2): 209-212.
Fowler, V. G., Jr., G. Sakoulas, et al. (2004). "Persistent bacteremia due to methicillin-resistant Staphylococcus aureus infection is associated with agr dysfunction and low-level in vitro resistance to thrombin-induced platelet microbicidal protein." J Infect Dis 190(6): 1140-1149.
Goerke, C., S. Campana, et al. (2000). "Direct quantitative transcript analysis of the agr regulon of Staphylococcus aureus during human infection in comparison to the expression profile in vitro." Infect Immun 68(3): 1304-1311.
Hawkins, C., J. Huang, et al. (2007). "Persistent Staphylococcus aureus bacteremia: an analysis of risk factors and outcomes." Arch Intern Med 167(17): 1861-1867.
Kornblum, J. S., S. J. Projan, et al. (1988). "A rapid method to quantitate non-labeled RNA species in bacterial cells." Gene 63(1): 75-85.
Kreger, A. S., K. S. Kim, et al. (1971). "Purification and properties of staphylococcal delta hemolysin." Infect Immun 3(3): 449-465.
Otto, M. (2010). "Basis of virulence in community-associated methicillin-resistant Staphylococcus aureus." Annu Rev Microbiol 64: 143-162.
Otto, M. and F. Gotz (2000). "Analysis of quorum sensing activity in staphylococci by RP-HPLC of staphylococcal delta-toxin." Biotechniques 28(6): 1088, 1090, 1092, 1096.
Paizs, B. and S. Suhai (2005). "Fragmentation pathways of protonated peptides." Mass Spectrom Rev 24(4): 508-548.
Rose, W. E., M. J. Rybak, et al. (2007). "Correlation of vancomycin and daptomycin susceptibility in Staphylococcus aureus in reference to accessory gene regulator (agr) polymorphism and function." J Antimicrob Chemother 59(6): 1190-1193.
Sakoulas, G., G. M. Eliopoulos, et al. (2005). "Reduced susceptibility of Staphylococcus aureus to vancomycin and platelet microbicidal protein correlates with defective autolysis and loss of accessory gene regulator (agr) function." Antimicrob Agents Chemother 49(7): 2687-2692.
Sakoulas, G., G. M. Eliopoulos, et al. (2002). "Accessory gene regulator (agr) locus in geographically diverse Staphylococcus aureus isolates with reduced susceptibility to vancomycin." Antimicrob Agents Chemother 46(5): 1492-1502.
Sakoulas, G., H. S. Gold, et al. (2006). "Effects of prolonged vancomycin administration on methicillin-resistant Staphylococcus aureus (MRSA) in a patient with recurrent bacteraemia." J Antimicrob Chemother 57(4): 699-704.
Schweizer, M. L., J. P. Furuno, et al. (2011). "Increased mortality with accessory gene regulator (agr) dysfunction in Staphylococcus aureus among bacteremic patients." Antimicrob Agents Chemother 55(3): 1082-1087.
Somerville, G. A., A. Cockayne, et al. (2003). "Synthesis and deformylation of Staphylococcus aureus delta-toxin are linked to tricarboxylic acid cycle activity." J Bacteriol 185(22): 6686-6694.
Szabados, F., K. Becker, et al. (2011). "The matrix-assisted laser desorption/ionisation time-of-flight mass spectrometry (MALDI-TOF MS)-based protein peaks of 4448 and 5302 Da are not associated with the presence of Panton-Valentine leukocidin." Int J Med Microbiol 301(1): 58-63.
Tholey, A. and E. Heinzle (2006). "Ionic (liquid) matrices for matrix-assisted laser desorption/ionization mass spectrometry-applications and perspectives." Anal Bioanal Chem 386(1): 24-37.
Traber, K. and R. Novick (2006). "A slipped-mispairing mutation in AgrA of laboratory strains and clinical isolates results in delayed activation of agr and failure to translate delta- and alpha-haemolysins." Mol Microbiol 59(5): 1519-1530.
Traber, K. E., E. Lee, et al. (2008). "agr function in clinical Staphylococcus aureus isolates." Microbiology 154(Pt 8): 2265-2274.
Tsuji, B. T., R. D. Maclean, et al. (2011). "Impact of accessory gene regulator (agr) dysfunction on vancomycin pharmacodynamics among Canadian community and health-care associated methicillin-resistant Staphylococcus aureus." Ann Clin Microbiol Antimicrob 10: 20.
Turner, W. H. (1978). "Purification and characterization of an immunologically distinct delta-hemolysin from a canine strain of Staphylococcus aureus." Infect Immun 20(2): 485-494.
Verdon, J., N. Girardin, et al. (2009). "delta-hemolysin, an update on a membrane-interacting peptide." Peptides 30(4): 817-823.
Vuong, C., H. L. Saenz, et al. (2000). "Impact of the agr quorum-sensing system on adherence to polystyrene in Staphylococcus aureus." J Infect Dis 182(6): 1688-1693.
Welker, M. and E. R. Moore (2011). "Applications of whole-cell matrix-assisted laser-desorption/ionization time-of-flight mass spectrometry in systematic microbiology." Syst Appl Microbiol 34(1): 2-11.
Wertheim, H. F., D. C. Melles, et al. (2005). "The role of nasal carriage in Staphylococcus aureus infections." Lancet Infect Dis 5(12): 751-762.

## Revendications

1. Procédé d'étude d'un échantillon contenant une population bactérienne de *Staphylococcus aureus* par une technique de spectrométrie de masse comprenant les étapes suivantes :
a) Disposer l'échantillon en contact avec un milieu permettant l'ionisation par action d'un rayon laser des molécules présentes dans l'échantillon,
b) Ioniser les molécules présentes dans l'échantillon grâce à un rayon laser,
c) Accélérer les molécules ionisées obtenues grâce à une différence de potentiel,
d) Laisser se déplacer librement les molécules ionisées et accélérées dans au moins un tube sous pression réduite,
e) Détecter au moins une partie des molécules ionisées et accélérées s'étant déplacées librement, de manière à mesurer le temps qu'elles ont mis pour parcourir au moins un tube sous pression réduite et à obtenir un signal correspondant au nombre de molécules ionisées détectées à un instant donné,
f) Calculer le rapport masse sur charge [m/z] des molécules détectées, de manière à obtenir un signal correspondant au nombre de molécules ionisées d'une même masse sur charge [m/z], en fonction du rapport m/z des molécules détectées,
g) Déterminer, de manière directe ou indirecte, si parmi les molécules ionisées obtenues à l'étape b), il y avait ou pas des molécules ionisées de masse/charge [m/z] égale à 3005 ± 5 Th ou égale à 3035 ± 5 Th,
h) Emettre une décision conditionnée par le résultat obtenu à l'étape g).

2. Procédé selon la revendication 1 **caractérisé en ce que** l'étape h) correspond à une étape de corrélation de l'absence à la fois de molécules ionisées de m/z égale à 3005 ± 5 Th et de molécules ionisées de m/z égale à 3035 ± 5 Th avec l'absence d'hémolysine δ et de son variant G10S dans la population bactérienne de *Staphylococcus aureus* analysée.

3. Procédé selon la revendication 1 **caractérisé en ce que** l'étape h) correspond à une étape de corrélation de l'absence à la fois de molécules ionisées de m/z égale à 3005 ± 5 Th et de molécules ionisées de m/z égale à 3035 ± 5 Th avec un dysfonctionnent du système agr (« *Accessory Gene Regulator* ») chez la population bactérienne de *Staphylococcus aureus* analysée.

4. Procédé selon la revendication 1 **caractérisé en ce que** la population bactérienne de *Staphylococcus aureus* analysée provient d'un échantillon biologique d'un patient et **en ce que** l'étape h) correspond à une étape de corrélation de l'absence à la fois de molécules ionisées de m/z égale à 3005 ± 5 Th et de molécules ionisées de m/z égale à 3035 ± 5 Th avec l'émission pour ledit patient, d'un diagnostic clinique connu pour être lié au dysfonctionnement du système agr.

5. Procédé selon la revendication 1 ou 4 **caractérisé en ce que** la colonie bactérienne de *Staphylococcus aureus* analysée provient d'un échantillon biologique d'un patient et **en ce que** l'étape h) correspond à une étape de corrélation de l'absence à la fois de molécules ionisées de m/z égale à 3005 ± 5 Th et de molécules ionisées de m/z égale à 3035 ± 5 Th avec le diagnostic d'une infection chronique chez le patient.

6. Procédé selon la revendication 1 ou 4 **caractérisé en ce que** l'étape h) correspond à une étape de corrélation de l'absence à la fois de molécules ionisées de m/z égale à 3005 ± 5 Th et de molécules ionisées de m/z égale à 3035 ± 5 Th avec un classement de la population bactérienne *Staphylococcus aureus* analysée comme risquant de présenter une sensibilité diminuée aux glycopeptides (*i.e.* GISA).

7. Procédé selon l'une des revendications précédentes **caractérisé en ce que** la spectrométrie de masse est réalisée sur un échantillon contenant une population de 10 à 10⁹ bactéries, et de préférence de 10⁵ à 10⁶ bactéries.

8. Procédé selon l'une des revendications précédentes **caractérisé en ce que** la spectrométrie de masse utilisée est la spectrométrie de masse (SM) par désorption-ionisation assistée par matrice et mesure du temps de vol (MALDI-TOF).

9. Procédé selon la revendication 8 **caractérisé en ce que** la détermination de l'étape g) est directement déduite de la présence sur le spectre de masse obtenu d'un pic à une valeur de m/z égale à 3005 ± 5 Th ou à 3035 ± 5 Th ou de l'absence de pics à la fois à 3005 ± 5 Th et à 3035 ± 5 Th.

10. Procédé selon l'une des revendications 1 à 7 **caractérisé en ce que** la spectrométrie de masse utilisée est la MALDI TOF/TOF.

11. Procédé selon la revendication 10 **caractérisé en ce qu'**à l'étape d), on laisse se déplacer les molécules ionisées dans un premier tube sous pression réduite, on sélectionne les ions de masse m/z égale à 3005 ± 5 Th et/ou de masse m/z égale à 3035 ± 5 Th, on accélère à nouveau les molécules ionisées après leur fragmentation, et on laisse se déplacer librement dans un deuxième tube les molécules ionisées fragmentées ou non.

12. Procédé selon l'une des revendications 10 ou 11 **caractérisé en ce que** la détermination de l'étape g) est indirectement déduite de la présence d'au moins cinq fragments parmi les ions fragments y et b suivants :
- les ions fragments y de masse 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2005.148, 2118.232, 2219.280, 2306.312, 2419.396, 2532.480, 2647.507, 2775.565, 2846.603 et 2976.635 ; et les ions fragments b de masse 131.040, 202.077, 330.136, 445.163, 558.247, 671.331, 758.363, 859.410, 972.494, 1029.516, 1144.543, 1257.627, 1356.695, 1484.790, 1670.870, 1783.954, 1897.038, 2012.065, 2113.112, 2212.181, 2326.224, 2454.319, 2601.387, 2702.435, 2830.530 et 2958.625, avec une tolérance de ± 1.5 Th, pour le pic à 3005 ± 5 Th, et/ou
- les ions fragments y de masse 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2035.159, 2148.243, 2249.290, 2336.322, 2449.406, 2562.491, 2677.517, 2805.576, 2876.613 et 3006.646 ; et les ions fragments b de masse 131.040, 202.077, 330.136, 445.163, 558.247, 671.331, 758.363, 859.410, 972.494, 1059.526, 1174.553, 1287.638, 1386.706, 1514.801, 1700.880, 1813.964, 1927.048, 2042.075, 2143.123, 2242.191, 2356.234, 2484.329, 2631.398, 2732.445, 2860.540 et 2988.635, avec une tolérance de ± 1.5 Th, pour le pic à 3035 ± 5 Th.

13. Procédé selon l'une des revendications 1 à 12 **caractérisé en ce que** le milieu avec lequel l'échantillon est mis en contact correspond à une matrice adaptée à la SM MALDI déposée sur une cible, et **en ce que** le procédé contient avant l'étape b), l'obtention de la cristallisation de la matrice sur ou dans laquelle les molécules de l'échantillon sont adsorbées.

14. Procédé selon la revendication 13 **caractérisé en ce que** la matrice contient un composé choisi parmi l'acide 3,5-diméthoxy-4-hydroxycinnamique, l'acide α-cyano-4-hydroxycinnamique, l'acide férulique et l'acide 2,5-dihydroxybenzoïque.

## Patentansprüche

1. Verfahren zur Untersuchung einer eine Bakterienpopulation von *Staphylococcus aureus* enthaltenden Probe mittels einer Massenspektrometrie-Technik, umfassend die folgenden Schritte:
a) Anordnen der Probe in Kontakt mit einem Medium, das die Ionisierung der in der Probe vorhandenen Moleküle durch Wirkung eines Laserstrahls ermöglicht,
b) Ionisieren der in der Probe vorhandenen Moleküle mittels eines Laserstrahls,
c) Beschleunigen der erhaltenen ionisierten Moleküle mittels einer Potentialdifferenz,
d) die ionisierten und beschleunigten Moleküle sich in wenigstens einer Röhre unter reduziertem Druck frei bewegen lassen,
e) Erfassen wenigstens eines Teils der ionisierten und beschleunigten Moleküle, die sich frei bewegt haben, um die Zeit zu messen, die sie benötigt haben, um wenigstens eine Röhre unter vermindertem Druck zu durchlaufen, und um ein Signal zu erhalten, das der Anzahl von zu einem gegebenen Zeitpunkt erfassten ionisierten Molekülen entspricht,
f) Berechnen des Masse-zu-Ladung-Verhältnisses [m/z] der erfassten Moleküle, um ein Signal zu erhalten, das der Anzahl von ionisierten Molekülen mit einer gleichen Masse zu Ladung [m/z], in Abhängigkeit von dem m/z-Verhältnis der erfassten Moleküle entspricht,
g) direktes oder indirektes Bestimmen, ob es unter den bei Schritt b) erhaltenen ionisierten Molekülen ionisierte Moleküle mit einer Masse/Ladung [m/z] gleich 3005 ± 5 Th oder gleich 3035 ± 5 Th gab oder nicht,
h) Aussprechen einer durch das bei Schritt g) erzielte Ergebnis bedingten Entscheidung.

2. Verfahren nach Anspruch 1, **dadurch gekennzeichnet, dass** Schritt h) einem Schritt zur Korrelation der Abwesenheit sowohl von ionisierten Molekülen mit m/z gleich 3005 ± 5 Th als auch von ionisierten Molekülen mit m/z gleich 3035 ± 5 Th mit der Abwesenheit von δ-Hämolysin und seiner Variante G10S in der analysierten Bakterienpopulation von *Staphylococcus aureus* entspricht.

3. Verfahren nach Anspruch 1, **dadurch gekennzeichnet, dass** Schritt h) einem Schritt zur Korrelation der Abwesenheit sowohl von ionisierten Molekülen mit m/z gleich 3005 ± 5 Th als auch von ionisierten Molekülen mit m/z gleich 3035 ± 5 Th mit einer Fehlfunktion des agr-Systems ("Accessory Gene Regulator") bei der analysierten Bakterienpopulation von *Staphylococcus aureus* entspricht.

4. Verfahren nach Anspruch 1, **dadurch gekennzeichnet, dass** die analysierte Bakterienpopulation von *Staphylococcus aureus* von einer biologischen Probe eines Patienten stammt und dass Schritt h) einem Schritt zur Korrelation der Abwesenheit sowohl von ionisierten Molekülen mit m/z gleich 3005 ± 5 Th als auch von ionisierten Molekülen mit m/z gleich 3035 ± 5 Th mit dem Stellen einer klinischen Diagnose für den Patienten, die dafür bekannt ist, mit der Fehlfunktion des agr-Systems verbunden zu sein, entspricht.

5. Verfahren nach Anspruch 1 oder 4, **dadurch gekennzeichnet, dass** die analysierte Bakterienkolonie von *Staphylococcus aureus* von einer biologischen Probe eines Patienten stammt und dass Schritt h) einem Schritt zur Korrelation der Abwesenheit sowohl von ionisierten Molekülen mit m/z gleich 3005 ± 5 Th als auch von ionisierten Molekülen mit m/z gleich 3035 ± 5 Th mit der Diagnose einer chronischen Infektion bei dem Patienten entspricht.

6. Verfahren nach Anspruch 1 oder 4, **dadurch gekennzeichnet, dass** Schritt h) einem Schritt zur Korrelation der Abwesenheit sowohl von ionisierten Molekülen mit m/z gleich 3005 ± 5 Th als auch von ionisierten Molekülen mit m/z gleich 3035 ± 5 Th mit einer Einstufung der analysierten Bakterienpopulation von *Staphylococcus aureus* als Gefahr laufend, eine verminderte Empfindlichkeit gegenüber Glykopeptiden (d.h. GISA) aufzuweisen, entspricht.

7. Verfahren nach einem der vorhergehenden Ansprüche, **dadurch gekennzeichnet, dass** die Massenspektrometrie an einer Probe, die eine Population von 10 bis 10⁹ Bakterien und vorzugsweise von 10⁵ bis 10⁶ Bakterien enthält, durchgeführt wird.

8. Verfahren nach einem der vorhergehenden Ansprüche, **dadurch gekennzeichnet, dass** die eingesetzte Massenspektrometrie die Massenspektrometrie (MS) durch Matrix-unterstützte Desorption/Ionisation und Flugzeitmessung (MALDI-TOF) ist.

9. Verfahren nach Anspruch 8, **dadurch gekennzeichnet, dass** die Bestimmung des Schrittes g) direkt aus der Anwesenheit eines Peaks bei einem Wert von m/z gleich 3005 ± 5 Th oder bei 3035 ± 5 Th an dem erhaltenen Massenspektrum oder aus der Abwesenheit von Peaks sowohl bei 3005 ± 5 Th als auch bei 3035 ± 5 Th abgeleitet wird.

10. Verfahren nach einem der Ansprüche 1 bis 7, **dadurch gekennzeichnet, dass** die eingesetzte Massenspektrometrie die MALDI TOF/TOF ist.

11. Verfahren nach Anspruch 10, **dadurch gekennzeichnet, dass** man bei Schritt d) die ionisierten Moleküle sich in einer ersten Röhre unter vermindertem Druck bewegen lässt, die Ionen der Masse m/z gleich 3005 ± 5 Th und/oder der Masse m/z gleich 3035 ± 5 Th auswählt, die ionisierten Moleküle nach ihrer Fragmentierung erneut beschleunigt und man die fragmentierten oder nicht fragmentierten ionisierten Moleküle sich in einer zweiten Röhre frei bewegen lässt.

12. Verfahren nach einem der Ansprüche 10 oder 11, **dadurch gekennzeichnet, dass** die Bestimmung des Schrittes g) indirekt aus der Anwesenheit von wenigstens fünf Fragmenten unter den folgenden Fragment-Ionen y und b abgeleitet ist:
- den Fragment-Ionen y der Masse 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2005.148, 2118.232, 2219.280 , 2306.312, 2419.396, 2532.480, 2647.507, 2775.565, 2846.603 und 2976.635; und den Fragment-Ionen b der Masse 131.040, 202.077, 330.136, 445.163, 558.247, 671.331, 758.363, 859.410, 972.494, 1029.516, 1144.543, 1257.627, 1356.695, 1484.790, 1670.870, 1783.954, 1897.038, 2012.065, 2113.112, 2212.181, 2326.224, 2454.319, 2601.387, 2702.435, 2830.530 und 2958.625, mit einer Toleranz von ± 1,5 Th, für den Peak bei 3005 ± 5 Th, und/oder
- den Fragment-Ionen y der Masse 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2035.159, 2148,243, 2249.290, 2336.322, 2449.406, 2562.491, 2677.517, 2805.576, 2876.613 und 3006,646; und den Fragment-Ionen b der Masse 131.040, 202.077, 330.136, 445.163, 558.247, 671.331, 758.363, 859.410, 972.494, 1059.526, 1174.553, 1287.638, 1386.706, 1514.801, 1700.880, 1813.964, 1927.048, 2042.075, 2143.123, 2242.191, 2356.234, 2484.329, 2631.398, 2732.445, 2860.540 und 2988.635, mit einer Toleranz von ± 1,5 Th, für den Peak bei 3035 ± 5 Th.

13. Verfahren nach einem der Ansprüche 1 bis 12, **dadurch gekennzeichnet, dass** das Medium, mit dem die Probe in Kontakt gebracht wird, einer für die MALDI-MS ausgelegten, auf ein Target aufgebrachten Matrix entspricht und dass das Verfahren vor Schritt b) das Erreichen der Kristallisation der Matrix, an oder in der die Moleküle der Probe adsorbiert sind, enthält.

14. Verfahren nach Anspruch 13, **dadurch gekennzeichnet, dass** die Matrix eine Verbindung, ausgewählt aus 3,5-Dimethoxy-4-hydroxyzimtsäure, α-Cyano-4-hydroxyzimtsäure, Ferulasäure und 2,5-Dihydroxybenzoesäure, enthält.

## Claims

1. A method for studying a sample containing a bacterial population of *Staphylococcus aureus* by a mass spectrometry technique comprising the following steps:
a) placing the sample in contact with the medium allowing ionization by action of a laser beam of the molecules present in the sample,
b) ionizing the molecules present in the sample by means of a laser beam,
c) accelerating the ionized molecules obtained by a potential difference,
d) letting the ionized and accelerated molecules freely move in at least one tube under reduced pressure,
e) detecting at least one portion of the ionized and accelerated molecules which have freely moved, so as to measure the time they took for covering at least one tube under reduced pressure and for obtaining a signal corresponding to the number of detected ionized molecules at a given instant,
f) calculating the mass over charge ratio [m/z] of the detected molecules, so as to obtain a signal corresponding to the number of ionized molecules of a same mass over charge ratio [m/z], depending on the m/z ratio of the detected molecules,
g) determining either directly or indirectly whether the ionized molecules obtained in step b), there were or not ionized molecules of a mass/charge ratio [m/z] equal to 3005 ± 5 Th or equal to 3035 ± 5 Th,
h) issuing a decision conditioned by the results obtained in step g).

2. The method according to claim 1, **characterized in that** step h) corresponds to a step of correlation of the absence of both ionized molecules with m/z equal to 3005 ± 5 Th and ionized molecules of m/z equal to 3035 ± 5 Th with the absence of hemolysin δ and of its variant G10S in the analyzed bacterial population of *Staphylococcus aureus.*

3. The method according to claim 1, **characterized in that** the step h) corresponds to a step of correlation of the absence of both ionized molecules with m/z equal to 3005 ± 5 Th and ionized molecules of m/z equal to 3035 ± 5 Th with a dysfunction of the agr (« *Accessory Gene Regulator »)* system in the analyzed bacterial population of *Staphylococcus aureus*.

4. The method according to claim 1, **characterized in that** the analyzed bacterial population of *Staphylococcus aureus* comes from a biological sample from a patient and **in that** step h) corresponds to a step for correlation of the absence of both ionized molecules with m/z equal to 3005 ± 5 Th and ionized molecules of m/z equal to 3035 ± 5 Th with issuance for said patient of a clinical diagnostic known to be related to the dysfunction of the agr system.

5. The method according to claim 1 or 4, **characterized in that** the analyzed bacterial colony of *Staphylococcus aureus* stems from a biological sample from a patient and **in that** step h) corresponds to a step for correlation of the absence of both ionized molecules with m/z equal to 3005 ± 5 Th and ionized molecules of m/z equal to 3035 ± 5 Th with the diagnostic of a chronic infection in a patient.

6. The method according to claim 1 or 4, **characterized in that** step h) corresponds to a step for correlation of the absence of both ionized molecules of m/z equal to 3005 ± 5 Th and ionized molecules of m/z equal to 3035 ± 5 Th with a classification of the analyzed bacterial *Staphylococcus aureus population* as having the risk of exhibiting reduced sensitivity to glycopeptides (*i.e.* GISA).

7. The method according to one of the preceding claims **characterized in that** mass spectrometry is carried out on a sample containing a population from 10 to 10⁹ bacteria, and preferably from 10⁵ to 10⁶ bacteria.

8. The method according to one of the preceding claims, **characterized in that** the mass spectrometry used is mass spectrometry (MS) with matrix-assisted desorption-ionization and measurement of the time of flight (MALDI-TOF).

9. The method according to claim 8 **characterized in that** the determination of step g) is directly inferred from the presence on the obtained mass spectrum of a peak with an m/z value equal to 3005 ± 5 Th or to 3035 ± 5 Th or from the absence of peaks both at 3005 ± 5 Th and at 3035 ± 5 Th.

10. The method according to one of claims 1 to 7, **characterized in that** the mass spectrometry used is MALDI TOF/TOF mass spectrometry.

11. The method according to claim 10, **characterized in that** in step d), the ionized molecules are left to move in a first tube under reduced pressure, the ions with an m/z mass equal to 3005 ± 5 Th and/or with a m/z mass equal to 3035 ± 5 Th are selected, the ionized molecules after their fragmentation are again accelerated, and the, either fragmented or not, ionized molecules are left to freely move in a second tube.

12. The method according to one of claims 10 or 11, **characterized in that** the determination of step g) is indirectly inferred from the presence of at least five fragments from the following ion fragments y and b:
- the ion fragments y with masses 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2005.148, 2118.232, 2219.280, 2306.312, 2419.396, 2532.480, 2647.507, 2775.565, 2846.603 and 2976.635; and ion fragments b of masses 131.040, 202.077, 330.136, 445.163, 558.247, 671.331, 758.363, 859.410, 972.494, 1029.516, 1144.543, 1257.627, 1356.695, 1484.790, 1670.870, 1783.954, 1897.038, 2012.065, 2113.112, 2212.181, 2326.224, 2454.319, 2601.387, 2702.435, 2830.530 and 2958.625, with a tolerance of ± 1.5 Th, for the peak at 3005 ± 5 Th, and/or
- the ion fragments y of masses 147.113, 275.208, 376.255, 523.324, 651.419, 765.462, 864.530, 965.578, 1080.605, 1193.689, 1306.773, 1492.852, 1620.947, 1720.016, 1833.100, 1948.127, 2035.159, 2148.243, 2249.290, 2336.322, 2449.406, 2562.491, 2677.517, 2805.576, 2876.613 and 3006.646; and the ion fragments b of masses 131.040, 202.077, 330.136, 445.163, 558.247, 671.331, 758.363, 859.410, 972.494, 1059.526, 1174.553, 1287.638, 1386.706, 1514.801, 1700.880, 1813.964, 1927.048, 2042.075, 2143.123, 2242.191, 2356.234, 2484.329, 2631.398, 2732.445, 2860.540 and 2988.635, with a tolerance of ± 1.5 Th, for the peak at 3035 ± 5 Th.

13. The method according to one of claims 1 to 12, **characterized in that** the medium with which the sample is put into contact corresponds to a matrix suitable for MALDI MS deposited on a target, and **in that** the method includes before step b), the obtaining of crystallization of the matrix on or in which the molecules of the sample are adsorbed.

14. The method according to claim 13, **characterized in that** the matrix contains a compound selected from 3,5-dimethoxy-4-hydroxycinnamic acid, α-cyano-4-hydroxycinnamic acid, ferulic acid and 2,5-dihydroxybenzoic acid.
